# EUROPEAN PATENT APPLICATION

(11) **EP 1 479 381 A1**
(43) Date of publication of application: **24.11.2004**
(21) Application number: 03011029.0
(22) Date of filing: 19.05.2003
(51) Int. Cl.: A61K 9/20

(54) **Pharmaceutical dosage form comprising a solid solution**

(71) Applicant: EURO-CELTIQUE S.A., 2330 Luxembourg (LU)
(72) Inventor: Rein, Hubert, 35396 Giessen-Wieseck (DE); Steffens, Klaus-Jürgen, 53359 Rheinbach-Flerzheim (DE)
(74) Representative: Maiwald Patentanwalts GmbH

(57) **Abstract**

The invention concerns a method for producing of a pharmaceutical dosage form being a solid solution wherein the dosage form is produced by extrusion of an overwet mixture of at least one pharmaceutically active compound and at least one polysaccharide.

## Description

The present invention concerns a pharmaceutical dosage form comprising a solid solution, wherein the dosage form comprises a pharmaceutically active compound in a polysaccharide matrix.

The present invention also concerns a method of producing a pharmaceutical dosage form comprising a solid solution wherein the dosage form is produced by extrusion of an overwet mixture of at least one pharmaceutically active compound and at least one polysaccharide. The present invention is particularly concerned with a pharmaceutical dosage form being a solid solution wherein the dosage form comprises a pharmaceutically active compound in a starch matrix.

Extrusion is a widespread production process exercised in different fields of technology. It is e.g. used in the adhesive industry or in plastics processing for modifying polysaccharides, in particular starchs. Extrusion is also known in food technology for the production of starch-containing compositions, such as noodles, so-called peanut-flips or various sweets. The production conditions of all these processes are usually selected in such a way that foamed or "popped" products are obtained.

In pharmaceutical technology extrusion is commonly used for the production of matrix-based pharmaceutical dosage forms. A multitude of materials may be used for production of pharmaceutical dosage forms by extrusion. Thus extrusion technology may be used for the processing of waxes, fatty alcohols, fats and various thermoplastics and duroplastics. For example, extruded matrices from various polymer compounds are disclosed in EP-A2 0 240 904, EP-A2 0 240 906 and EP-A2 0 358 105.

The processing of starch-containing mixtures into pharmaceutical products (capsules) by means of an injection-molding technique is described in EP-B2 0 118 240.

One fundamental disadvantage of common extruded pharmaceutical dosage forms is in the need for excipients such as plastics, waxes or even fatty alcohols. These adjuvants, which are not biologically degradable and may be in part environmentally harmful, such as residual monomers of the polymers used, are, even up to the present day, often indispensable for the dosage forms produced by means of extrusion methods.

Accordingly the use of polysaccharides such as starchs as adjuvants is of particular importance and interest for the production of pharmaceutical dosage forms by extrusion, as they are biologically degradable and thus neither detrimental to the patients nor the environment.

Extrusion of biologically degradable polysaccharides such as starch has been described before. EP-A1 0 580 860 discloses a method for producing solid dispersions of an active agent in a polymeric carrier. Among the raw materials which may be used, starch and starch derivatives are mentioned in general. However, the method described therein is not suitable for preparations having starch as the main ingredient.

International patent application WO 00/64415 describes a method for producing a water-insoluble amorphous or partially amorphous controlled release matrix comprising a pharmaceutically active compound embedded in a starch matrix. The characterizing feature of these dosage forms is that they allow for a reproducible release behaviour of the active compounds from the starch matrix. From the examples it can be taken, that the starch matrices of WO 00/64415 are solid dispersions, which comprise the active compounds evenly distributed within the matrix and which release the active compounds in a sustained manner, as is typical for extruded matrices.

In pharmaceutical technology, orally administered dosage forms such as, tablets, pills or capsules are by far the most important. If produced by extrusion, these dosage forms usually are so-called controlled release (retard) dosage forms which contain a relatively high dose of active compound and release this active compound in a controlled manner over an extended period of time. For the patient, this means that the frequency of medication can be significantly reduced. From a medical-pharmacological point of view, the advantage of controlled release dosage forms lies in a very uniform concentration of the active agent in the blood, producing a long-lasting effect, often with reduced side-effects.

When formulating controlled release dosage forms, the so-called matrix form is of fundamental importance. Matrix means a shaped body, such as a granule, made from inert adjuvants, which releases the active compounds into the gastro-intestinal tract in a controlled manner.

A general advantage of the extrusion process is that pharmaceutical dosage forms can be produced in a continuous process which is usually more time-, energy- and thus cost-efficient than e.g. production by granulation.

However, controlled release is not always desired. For many applications, immediate release of the active agent may be preferred. Typical immediate release formulations may be solid dosage forms; generally, they are not, however, based on matrix formulations.

Thus it would be desirable to have pharmaceutical matrix-based dosage forms that are producible by extrusion and provide for fast or immediate release of the active compounds.

Under certain circumstances immediate release dosage forms may be particularly advantageous such as in pain therapy where sudden pain attacks have to be treated rapidly and efficiently. However, the active compounds of immediate release dosage forms are usually easy extractable from typical immediate release dosage forms, so that use of immediate release dosage forms is problematic in all cases where there is a danger of abuse, such as with opioid analgesics.

Thus there is a need for matrix-based pharmaceutical dosage forms which release the active compound(s) rapidly and from which the active compound may be extractable only with substantial effort.

There is a need for oral pharmaceutical dosage forms which ensure a substantially fast or immediate release of the active compound(s) from the matrix, wherein the matrix is formed from biologically degradable adjuvants.

Such dosage forms should also provide for good storage stability, as required by admission guidelines, government regulations etc.

It is an object of the instant invention to satisfy these needs in the prior art.

One object of the present invention is thus the provision of a pharmaceutical dosage form for fast or immediate release wherein the dosage form comprises at least one pharmaceutically active compound in a matrix. Another object of the present invention is a pharmaceutical dosage form as that mentioned above wherein the dosage form is produced by extrusion.

It is another object to provide such a dosage form based on fully biologically degradable adjuvants. It is yet another object to provide dosage forms with substantially reduced abuse potential for fast or immediate release of controlled substances, especially opioids and opiates.

These and other objectives as they may become clear from the description are attained by the independent claims. Preferred embodiments of the invention are defined by the dependent claims.

It has now surprisingly been found that pharmaceutical dosage forms can be produced which are solid solutions and comprise at least one pharmaceutically active compound in a polysaccharide matrix. It has moreover been surprisingly found that such pharmaceutical dosage forms being solid solutions can be produced by extrusion of an overwet mixture of at least one pharmaceutically active compound and at least one polysaccharide.

Such pharmaceutical dosage forms, which are solid solutions and comprise at least one pharmaceutically active compound in a polysaccharide matrix, provide numerous advantageous characteristics.

These pharmaceutical dosage forms comprise a stable, non-erosive, water-insoluble diffusion matrix which preferably provides for substantially fast or immediate release of the active compound(s) from the matrix while at the same time ensuring that the pharmaceutically active compound(s) can be extracted from the dosage form only with significant difficulties. A particular advantage of solid solutions is that they comprise the active compound in a solubilized state within the matrix so that the compound can be readily absorbed by the gastro-intestinal system, once it has been released from the matrix.

Another surprising feature of the pharmaceutical dosage forms according to the invention given that they comprise the active compound(s) in a dissolved state is their high stability over prolonged periods, such as one to three years, and preferably one to two years.

Another advantage of the inventive pharmaceutical dosage forms being solid solutions is that, depending on the production parameters, they may also provide for a sustained release of the active compound(s).

Thus, the present invention is also directed to a method of producing a pharmaceutical dosage form being a solid solution wherein the dosage form comprises at least one pharmaceutically active compound in a polysaccharide matrix. The present invention is also directed to methods of producing such pharmaceutical dosage forms by extrusion of an overwet mixture of at least one pharmaceutically active compound and at least one polysaccharide.

In a preferred aspect, the present invention is directed to pharmaceutical oral dosage forms as mentioned above wherein the matrix may be formed by different types of starchs.

The production of pharmaceutical dosage forms according to the present invention which are solid solutions and comprise at least one pharmaceutically active compound in a polysaccharide matrix, may generally be performed using the techniques as described in WO 00/64415, which is hereby incorporated by reference.

However, there is one important difference between the extrusion production process as described in WO 00/64415 and the current process. In contrast to the extrusion process described in WO 00/64415, extrusion according to the present invention is performed with an overwet mixture of at least one pharmaceutically active compound and at least one polysaccharide. Only if overwet mixtures of pharmaceutically active compounds and polysaccharides are extruded, pharmaceutical dosage forms are obtained which are solid solutions.

If in the context of the present invention, use is made of the term "overwet mixture", this refers to a mixture of at least one pharmaceutically active compound and at least one polysaccharide comprising at least the amount of water that is necessary to dissolve the at least one pharmaceutically active compound. The term "overwet mixture" also includes mixtures of at least one pharmaceutically active compound and at least one polysaccharide comprising an amount of water which is significantly higher than the amount necessary for dissolving the at least one pharmaceutically active compound.

Usually overwet mixtures according to the invention will comprise more than 15 % (w/w) water, more than 20 % (w/w), more than 25 % (w/w) and even more than 30 % (w/w) water, based on the weight of the overwet mixture comprising the at least one pharmaceutically active compound, at least one polysaccharide and other optional excipients.

The water content of the final dried and extruded pharmaceutical preparations may be significantly lower and be well below 15 % (w/w) water based on the weight of the final preparation.

In the context of the present invention the term "solid solution" refers to the situation where at least one pharmaceutically active compound is truly dissolved within the polysaccharide matrix. A true solution is characterized by a substantially homogenous distribution of solvatized single molecules of the dissolved component in the solvent. The dissolved component is, in the invention, formed by the active compound. The solvent is the polysaccharide matrix. The detection of the dissolved state of the pharmaceutically active compound within the polysaccharide matrix may be performed with the aid of different techniques.

By means of infrared spectroscopy (IR) the crystalline or partially crystalline state of the pharmaceutically active compound may be compared with the dissolved state within the matrix. If the peaks indicative of the crystalline or partially crystalline state of the pharmaceutically active compound can not be detected within the matrix, this may indicate that the pharmaceutically active compound is contained within the matrix in a molecularly dissolved state (see also examples). This technique can also be used to detect whether the polysaccharide is crystalline, or amorphous.

By means of differential scanning calorimetry (DSC) the transition of the crystalline or partially crystalline starch into the amorphous state can be detected. Under suitable extrusion conditions the starch becomes completely vitrified, i.e. changes into the amorphous state. After the extrusion is carried out according to the invention, no peaks in the decisive temperature range are detectable. This technique can also be used to detect dissolution of crystalline active compound.

The transition from the crystalline or partially crystalline into the amorphous or partially amorphous state may also be detected with the aid of x-ray diffraction. After application of the inventive method, the x-ray diffraction pattern of the corresponding extrudate does not have any peaks anymore which indicate a crystalline portion of the starch. X-ray diffraction analysis may be also be used for determining whether the pharmaceutically active compounds are dissolved within the matrix.

The analysis may alternatively be performed by (high resolution or broadline) NMR-measurements.

In the context of the present invention the terms "immediate release" or "substantially fast release" refer to the fact that pharmaceutical dosage forms according to the present invention do not provide for a sustained release of the active compound as that term is understood by the person skilled in the art. Particularly it refers to the fact that pharmaceutical preparations according to the present invention do not provide a sustained release, as do the pharmaceutical dosage forms comprising an extruded starch matrix as disclosed in WO 00/64415. Thus the terms "immediate release" and "fast release" refer to the situation that the pharmaceutical dosage forms of the present invention, being solid solutions, release the at least one active compound more rapidly than the pharmaceutical dosage forms to WO 00/64415, which are solid dispersions, no solid solutions.

Preferably, the inventive preparations will have substantially released the active compound(s) after 10, 9 or 8 hours, preferably after 7 hours, more preferably after 6 hours, even more preferably after 5 hours and most preferably after 4 hours.

A matrix-based pharmaceutical dosage form being a solid solution preferably provides a substantially fast release of the pharmaceutically active compound, whereby at least 50 % of the compound is released after 4 hours, preferably after 3 or 2 hours, more preferably after one hour and even more preferably after 30 minutes.

A solid solution matrix-based pharmaceutical dosage form preferably provides for a substantially fast or immediate release in that the preparation releases at least 75 % of the active compound after 10, 8 or 7 hours, preferably after 6 hours, more preferably after 5 hours, even more preferably after 4 hours, and most preferably after 3 hours.

As set out above pharmaceutical preparations in accordance with the invention preferably provide for a fast or immediate release of the active compounds.

However, in other embodiments, pharmaceutical preparations may be produced in accordance with the invention that provide for a sustained release of the active compounds. In these cases, the preparations release the active compounds completely after 8 to 12 hours, even more preferably after 12 to 24 hours. These embodiments have usefulness where fast or immediate release of the active compound is not important, or controlled (sustained) release is even desired. In such cases, other advantages of the invention may be useful, such as the abuse-prevention potential of the inventive formulations.

According to the present invention, the pharmaceutical dosage form being a solid solution is considered to be "hardly" extractable if the pharmaceutically active compound can be extracted from the pharmaceutical dosage form only with significant difficulties such as grinding the preparation with tools like a hammer which are not typically used by street junkies. Thus, preparations in accordance with the invention significantly impede the mechanical extractability of the active compound from the matrix. Hardly extractable pharmaceutical dosage forms according to the present invention are moreover characterised by substantial matrix stability towards acids, bases and other solvents. A starch matrix according to the invention e.g. neither dissolves in the acidic environment of the stomach nor in the neutral to basic environment of the intestine.

According to the invention "storage stable" or "storage stability" means that upon storage under standard conditions (at least two years at room temperature and usual humidity) the amounts of the active compounds of a medicament formulation do not deviate from the initial amounts by more than the values given in the specification or the guidelines of the common Pharmacopoeias. According to the invention, storage stability also means that a preparation produced according to the invention can be stored under standard conditions (60 % relative humidity, 25 °C) as it is required for admission to the market.

According to the present invention the term "matrix-based pharmaceutical dosage forms" defines those dosage forms that comprise the pharmaceutically active compound within a matrix, in the sense known to the person skilled in the art. The matrix-based pharmaceutical dosage forms may be coated with additional layers. Such layers may be formed by typical film-layer forming polymers such as polymers of the commercially available Eudragit®- or Surelease®-type. The coatings may also comprise an initial amount of the pharmaceutically active compound as well as typical pharmaceutical excipients such as colorants, odorants and conserving agents. Such coatings can, in some preferred embodiments, themselves provide a controlled release characteristic of the dosage form; alternatively, such coatings may just provide enteric protection in the stomach, or have a mechanically protective or even a merely decorative function.

In a further preferred embodiment, the matrix of the dosage form made according to the invention is amorphous or partially amorphous. In a further preferred embodiment, the invention relates to a matrix that comprises starch or a derivative thereof, in particular amorphous or partially amorphous starch or a derivative thereof, as the polysaccharide.

In this context it is pointed out that the degree of conversion of the polysaccharide into the glassy state is often decisive for the reproducibility of the release behaviour of the active agents. Release of the active agent is usually studied in a Paddle Aggregator Model according to Pharmacopeia Europeae in the liquids of 0.1 n HCl pH 1.0 (artificial gastric fluid) and Sörensen buffer pH 7.2 (artificial intestinal fluid). In this context it is again pointed out that the extrudates do not dissolve in the test, but nearly a swelling of the extrudates may be observed.

In the context of the present invention the term "pharmaceutically active compound" relates to common pharmaceutically active compounds which may be formulated into matrix-based dosage forms by common processes such as granulation or extrusion. Pharmaceutically active compounds according to the present invention preferably comprise analgesically active opioid agonists and opioid antagonists.

Thus, pharmaceutically active compounds of the present invention may comprise as opioid agonists e.g morphine, oxycodone, hydromorphone, propoxyphene, nicomorphine, dihydrocodeine, diamorphine, papaveretum, codeine, ethylmorphine, phenylpiperidine and derivates thereof, methadone, dextropropoxyphene, buprenorphine, pentazocine, tilidine, tramadol and hydrocodone.

Pharmaceutical dosage forms according to the present invention may also comprise as pharmaceutically active compounds opioid antagonists such as naltrexone, naloxone, nalmefene, nalorphine, nalbuphine, naloxoneazinen, methylnaltrexone, ketylcyclazocine, norbinaltorphimine, naltrindol, 6-ß-naloxol and 6-ß-naltrexol.

The active compounds may also be used in the form of their pharmaceutically acceptable and equally active derivatives such as the free base, salts and the like. Salt forms may comprise e.g. the hydrochloride, sulfate, bisulfate, tatrate, nitrate, citrate, bitatrate, phosphate, malate, maleate, hydrobromide, hydroiodide, fumarate or succinate salts of the active compound.

Further suitable pharmaceutically active compounds may be found e.g. in EP-A1 0 580 860.

According to the present invention the solid solution matrix of the pharmaceutical dosage forms may be produced by extrusion.

Extrusion may be performed as it is known in the art. Thus, extrusion may be performed using a single-screw extruder or a twin-screw extruder. Twin-screw extruders may comprise either counter- or co-rotating screws comprising optionally kneading elements. Preferred extruder types for single-screw extruders comprise e.g. a Brabender-extruder. Preferred twin-screw extruders comprise e.g. a Werner-Pfleiderer twin-screw extruder or a Leistritz Micro 18 twin-screw extruder.

The exact extrusion parameters can be easily determined by the person skilled in the art. They depend on the extruder type, the pharmaceutically active compound, the material used for preparation of the matrix as well as additional pharmaceutical excipients.

Extrusion parameters comprise parameters such as the heating temperature for the different extruder zones, the nozzle diameter and form, the rotating speed of the screw, the feeding rate, the water content of the preparation as well as the order of addition of the different components.

Preferred embodiments for specific extruders are given in the example section.

According to the present invention the solid solution matrix of the matrix-based pharmaceutical dosage forms comprises at least one polysaccharide as the matrix-forming polymer, at least one pharmaceutically active compound and optionally pharmaceutically acceptable excipients.

The pharmaceutically acceptable excipients comprise lubricants, plasticizers, colorants, conserving agents, viscosity-influencing agents, surfactant fillers etc.

Pharmaceutically acceptable excipients may therefore comprise lactose as filler, surfactants such as sodium lauryl sulphate, silicium dioxide, hydrophilic or amphiphilic liquids such as polyglycol, glycerol, polyethylene glycol, fatty acids, fats, saturated carbohydrates etc.

However, as it is well-known that these excipients may influence the release behaviour of the pharmaceutical dosage forms, care has to be taken that the excipients do not change the rapid or immediate release formulations of the present invention into sustained-release formulations. if immediate release formulations are to be provided. Thus, matrix-based pharmaceutical dosage forms being solid solutions that comprise only a polysaccharide as the matrix-forming polymer and at least one pharmaceutically active compound constitute a preferred embodiment of the invention.

The matrix-forming polymers (for example) may be based on common polymers such as cellulose, cellulose derivatives or starch. According to the invention the use of starch for forming the matrix is preferred. Especially preferred is the use of starchs such as tapioca-, potato-, corn- or wheat-starch. Commercially available starchs such as Starch 1500® (a com-starch distributed by Colocon), Waxilys® (a corn-starch distributed by Roquette) or Eurylon® 7 (a com-starch distributed by Roquette) are suitable. Other usable starch types comprise acetyl starch. A particularly preferred starch is corn starch. The above-mentioned starchs or derivatives thereof may also be mixed for forming a solid solution matrix-based pharmaceutical dosage form.

If for some particular reason there is no need for the matrix to consist of biological degradable polymers, other matrix-forming polymers such as polyvinyl pyrollidone, methacrylic polymers and other matrix-forming polymers or substances such as waxes which are well-known in the art may be used.

During the extrusion process, i.e. while using or applying heat, shear forces and pressure, an amorphous or partially amorphous matrix is generated from the crystalline or partly crystalline polysaccharide mixtures thereof containing at least one pharmaceutically active compound in a dissolved state.

For the reproducible production of dosage forms with reproducible release behaviour based on the extrusion of polysaccharides and derivatives thereof, the extrusion conditions, such as temperature, extruder geometry and extrusion speed are very important. For example, native starch may be completely plastified or vitrified under suitable extrusion conditions, so that homogeneous plastic-like shaped bodies are obtained that can be considered to be solid solutions as they contain the pharmaceutically active compound in a molecularly dissolved state.

When extruding starch, heating is usually only necessary at the beginning of the process. In the further course of the process, the heat generated by the strong shear and friction is preferably eliminated by cooling, in order to maintain a constant temperature. The formulations used in the inventive method are comprised of a mixture of polysaccharides and/or derivatives thereof, preferably of a mixture of starch and/or starch derivatives, there are being various types of starch which are suitable. Furthermore, the mixture contains at least one pharmaceutically effective substance in an amount of up to 50 % (w/w), preferably of up to 30 % (w/w), even more preferably of up to 20 % (w/w) and most preferably of up to 10 % (w/w) based on the total weight of the formulation and may additionally comprise further different substances. Water has to be included to ensure that an overwet pre-blend or mixture is obtained as defined above.

After thoroughly mixing under addition of water the obtained overwet pre-blend should preferably be screened so that it is free of all lumps, in order to ensure perfect conveyance through the screw feeder. When generating a matrix according to the inventive method, the temperature at the orifice of the extruder should not exceed 100 °C under normal pressure, since at temperatures above 100 °C the formation of a matrix free of pores will hardly be possible. The total energy fed into the process, in the form of shear-forces, temperature, heat or pressure, should be as constant as possible and should be sufficient to achieve glass transition. The optimal screw speed and geometry of the extruder may be adjusted to the mixture comprising the inventive carrier and water. At extrusion temperatures below 100 °C and suitable screw speeds, transparent and completely amorphous products are obtained in most cases. The degree of plastification of the active agent/polysaccharide mixtures and preferably of the active agent/starch mixtures is closely linked to the screw speed. While extrusion is no longer possible below a lower speed, a screw speed which is to high causes the dosage form to "pop open". Generally, parameters that lead to "popped" products should be avoided, as so-called "popped" or "foamed" products do not allowe for production of pharmaceutical dosage forms with a reducible release behaviour.

Extrusion may be performed with a single-screw extruder. One particularly preferred extruder is the Brabender extruder Type 811201 (Brabender, Duisburg, Germany). The screw of this extruder does typically not comprise compression elements and the extruder may be heated in three different zones, particularly at the feeder, the barrel and at the die segment. The screw may be run at speeds from 25 to 250 revolutions per minute (rpm) and the feeding rate may be 20 to 250 g/minute. The nozzles may provide for a diameter of 1 to 10 mm, preferably of 2 to 8 mm and more preferably of 3 to 5 mm.

Preferably, the screw speed during the extrusion process is between 90 to 180 rpm, more preferably between 100 and 150 rpm, and most preferably around 110, 120, 130, 140 or 150 rpm. The temperature at the feeder is typically between 30 to 100°C, preferably between 40 to 90°C, more preferably between 50 to 70°C, and even more preferably around 55, 60 and 65°C. The heating temperature at the barrels may be between 30 to 120°C, preferably between 40 to 90°C, more preferably between 50 to 80°C, and even more preferably approximately around 55, 60, 65, 70, 75 or 80°C. The temperature at the nozzle may be preferably between 80 to 120°C, more preferably between 90 to 100°C, and most preferably around 90 to 95°C. The nozzle diameter preferably is 2.5, 3.5 or 5 mm.

During extrusion with twin-extruders (such as Werner & Pfleiderer ZSK 25 or Leistritz Mikro 18), screw speeds should be between 120 to 250 rpm, preferably between 120 to 200 rpm, more preferably between 120 to 170 rpm, and most preferably around 150 rpm.

The temperature at the feeder may be between 20 to 80°C, preferably between 30 to 60°C, more preferably between 40 to 50°C, and even more preferably around 40, 45 or 50°C.

The temperature in the barrel heating zones may be between 30 to 180°C, between 40 to 160°C, between 60 to 130°C and between 70 to 100°C. Temperatures of approximately 40, 50, 60, 70, 75, 80, 115, 120, 125, 150, 155, 160°C are also preferred. Typically, the temperature of the heating zones of the barrel which are close to the nozzle will be lower than the temperature of those heating zones which are close to the feeder.

The temperature at the nozzle may be between 20 to 100°C, preferably between 50 to 95°C, and even more preferably around 90°C.

Dosage forms with immediate or fast release may be obtained for example by extrusion below the temperature of gelatinisation of the polysaccharides and preferably of the starchs used in each case.

The dosage forms produced according to the present invention may be used to produce granulates for tabletting and filling capsules, and adjuvants for direct tabletting, for further processing by means of extrusion molding techniques and/or as mono-block dosage forms, where the extrudate is formed by means of suitable arrangement at the extrusion die - similar to the case of food-production.

The invention allows the production of mono-block forms, in the process of which an extrudate is created that is vitrified only at the surface and includes the active compounds in a dissolved state in its interior.

In order to achieve the desired bio-pharmaceutical properties of the dosage forms, co-adjuvants known for producing solid forms may be used. Drying of the dosage form can take place nearly through the frictional heat that is created so that a following drying step is not necessary. In a production extruder, preferably a twin-screw extruder, especially preferred in a forced conveying twin-screw extruder, all process steps, such as dosing, moisturing, mixing, extruding and forming can be carried out continuously. The inventive method is thus able to combine the processes of mixing, granulating and drying for which no additional energy has to be expanded in one piece of equipment.

By the invention, also the number of possible incompatibilities is reduced since the dosage form may comprise for example only one excipient, preferably starch or a derivative thereof, and one pharmaceutically active substance. The matrix made according to the inventive method further prevents a possible unintended de-mixing.

The pharmaceutical dosage forms obtained through the inventive method comprise a matrix which is basically a non-erosive, water-insoluble diffusion matrix. It may however be partially swellable in water or aqueous buffers.

An examination of the course of the release of the active compound shows that the release of the active compound is preferably controlled by diffusion and follows the so-called lapidus rule, as shown in the tests described below. The course of the release does not change, even after storage of the dosage forms over several months.

It has been surprisingly been found that the dosage forms according to the present invention comprise the active compound in a dissolved state within a polysaccharide, preferably starch, matrix if extrusion is performed with an overwet mixture of the pharmaceutically active compound and the polysaccharide, preferably starch, even though the final product may comprise comparable water amounts to the solid dispersions disclosed in WO 00/64415 which provide for a sustained release. It has moreover been surprisingly found that the solid solutions according to the present invention do not significantly change their release behaviour even after prolonged storage for 24 months or more.

Thus, the present invention provides fast or immediate release pharmaceutical dosage forms which are based on a biological degradable matrix that comprises the active compound in a dissolved state so that the active compound may be efficiently absorbed by the gastro-intestinal system. Such pharmaceutical dosage forms provide also for long-extended storage stability. Due to the preferred immediate or substantially fast release of the active compound from the pharmaceutical dosage forms being solid solutions, pharmaceutical dosage forms of e.g. opioid analgesics may be provided that allow for counter-acting sudden pain attacks while at the same time reducing abuse-potential due to the poor mechanical extractability of the active compound from the dosage form. The dosage forms according to the present invention may preferably be formulated for oral uptake.

According to the invention the term "oral dosage forms" refers to dosage forms that are suitable for oral as well as peroral application as understood by the person skilled in the art. According to the invention peroral application refers to a situation where the dosage form has to pass the mouth, i.e. it has to be swallowed, before the pharmaceutically active compound is released (e.g. in the gastrointestinal system) while oral application refers to a situation where the pharmaceutically active compound is already released within the mouth cavity.

As illustrated in the examples, solid solutions according to the present invention provide for a lower glass transition temperature. Due to this lower glass transition temperatures, polysaccharide and preferably starch-based amorphous or partially amorphous matrix-forms can be produced at comparably low temperatures. This allows for the preparation of matrix-based dosage forms being solid solutions that comprise e.g. heat-labile pharmaceutically active compounds. In this way, substantially fast or immediate release (but also sustained release) pharmaceutical dosage forms of heat-labile pharmaceutically active substances can be produced by extrusion which is otherwise not possible.

Below, examples are given for the production of pharmaceutical dosage forms being solid solutions which comprise a pharmaceutically active compound within a polysaccharide matrix.

The preparations are produced by extrusion of different starch types together with naloxone as model pharmaceutically active substance. Pharmaceutical preparations with caffeine being solid dispersions were produced as comparative examples as described in WO 00/64415. The examples are meant to illustrate the invention and should by no means be interpreted in a restrictive manner.

### Example 1

Extrusion of solid solutions using a Leistritz Mikro 18 twin screw extruder

For the following experiments, four pharmaceutical dosage forms were extruded of which three contained naloxone HCl as a model substance while the fourth contained no active compound and served as a placebo-extrudate. The active compounds and the different starchs were mixed in a gravity mixer. The solid solutions were then produced under the following conditions.

| Starch | potato | tapioca | Waxilys 200 | tapioca |
|---|---|---|---|---|
| amount active compound temperature | 10 % | 10 % | 10 % | Placebo |
| feeder | | 51 °C | | |
| barrel | | 70 °C | | |
| nozzle | | 90 °C | | |
| screw rotating speed | | 150 rpm | | |
| water | | 480 g/h | | |

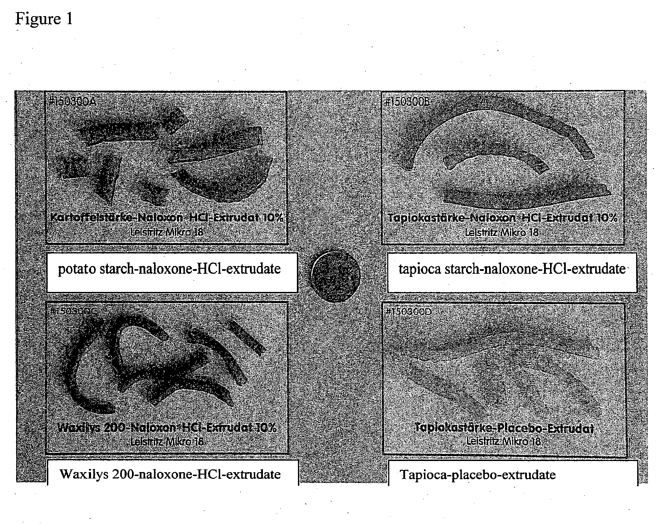

The outer appearance of the different extrudates is shown in Figure 1, the physical characteristics of the extruded solid solutions are shown in Table 1.

**Table I**

| Water | | Starch | extrusion speed | Pyknometer- density | T_{g} |
|---|---|---|---|---|---|
| pre-blend | extrudate | | | | |
| [%] | [%] | | [kg/h] | [g/ml] | [°C] |
| 33 | 7.4 | potato | 2.480 | 1.4205 | 78 |
| 27 | 9.9 | tapioca | 2.480 | 1.4765 | 84 |
| 28 | 8.1 | Waxilys 200 | 2.480 | 1.4795 | 90 |
| 29 | 9.9 | tapioca Placebo | 2.480 | 1.4356 | 98 |

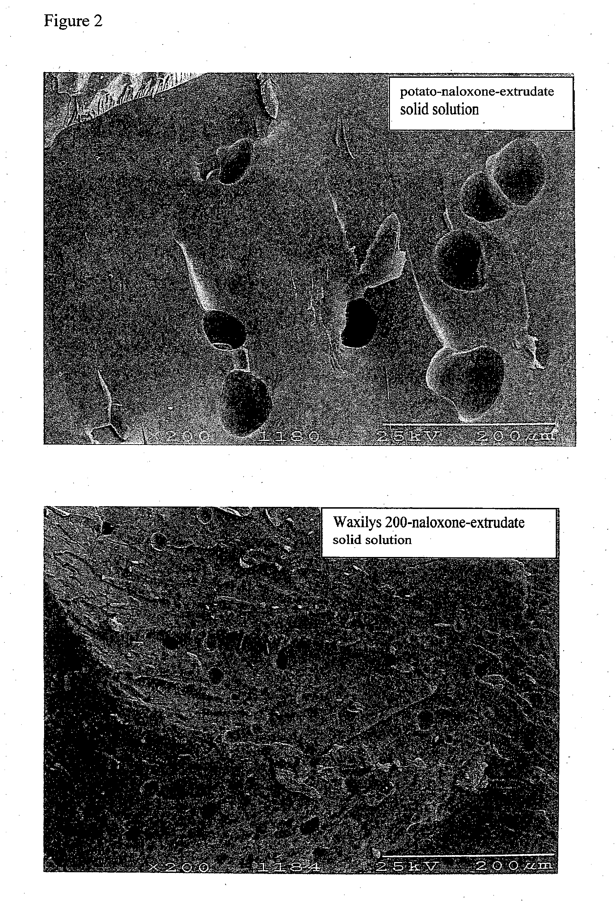

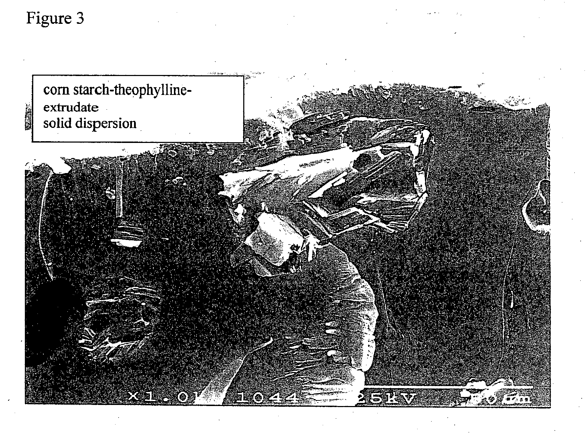

Figure 2 shows the appearance of extrudates being solid solutions based on potato starch or Waxilys 200 as determined by scanning electron microscopy at 200 fold magnification. For comparison, Figure 3 shows the appearance of solid dispersions of theophylline as determined with scanning electron microscopy at 1000 fold magnification. One can clearly see that solid solutions do not contain any crystals of the pharmaceutically active compound in comparison to the solid dispersions.

In the following the release behaviour of the solid solutions comprising naloxone in a potato starch matrix or in a Waxilys 200 matrix were determined according to the paddle method in 0.1 n HCI pH 1.0.

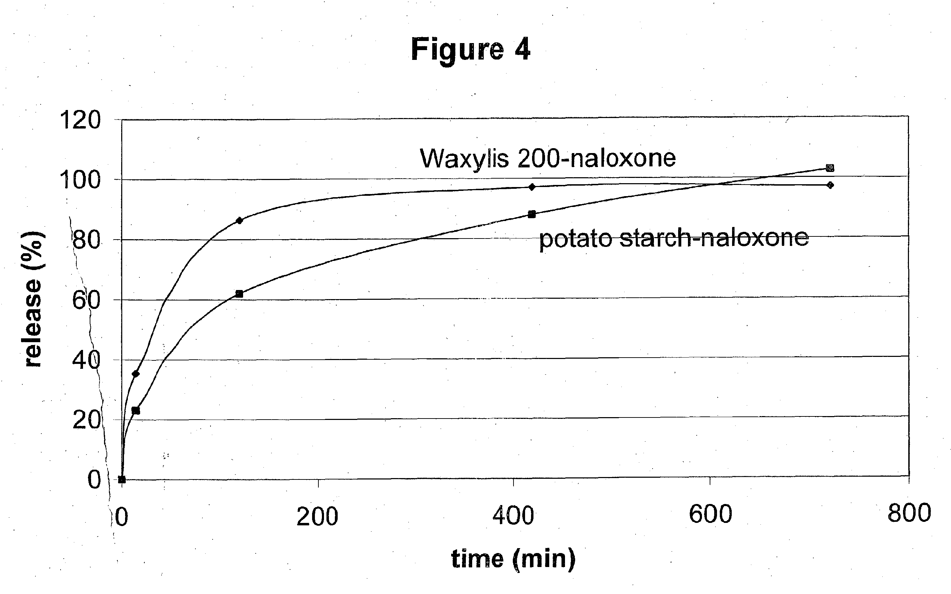

### The results are shown in figure 4 and table II:

**Table II:**

| time (min) | potato starci- naloxone % naloxone released | Waxilys 200 - naloxone % naloxone released |
|---|---|---|
| 15 | 35.3 | 23 |
| 120 | 86.3 | 62 |
| 420 | 97 | 87.9 |
| 720 | 97.1 | 102.8 |

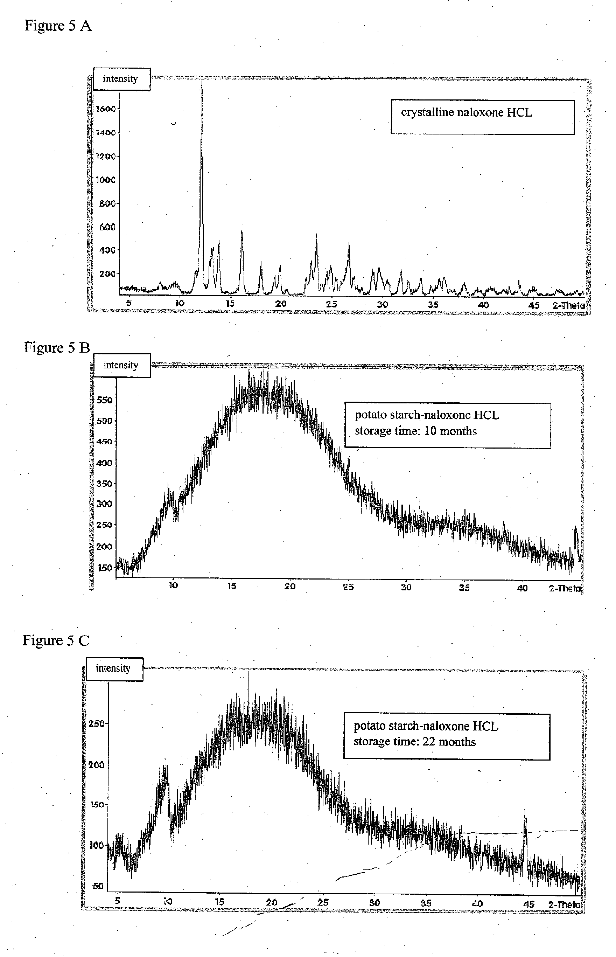

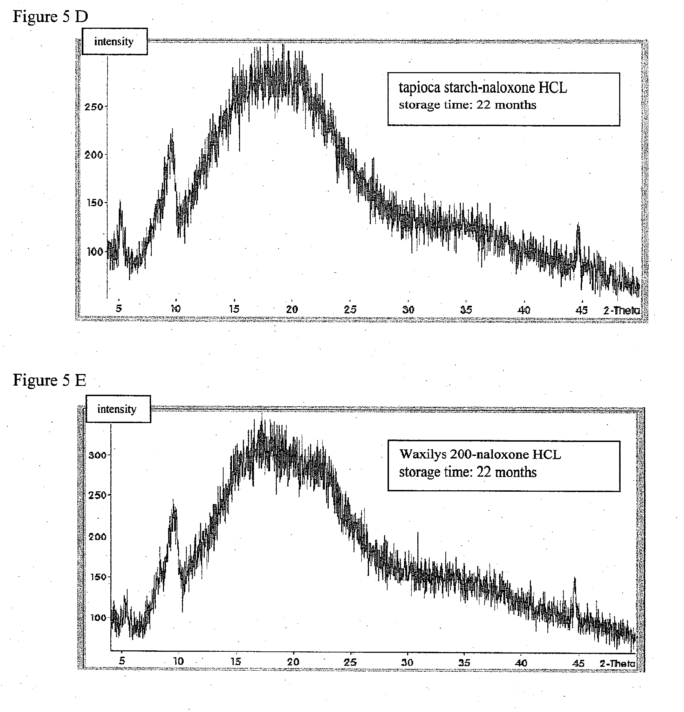

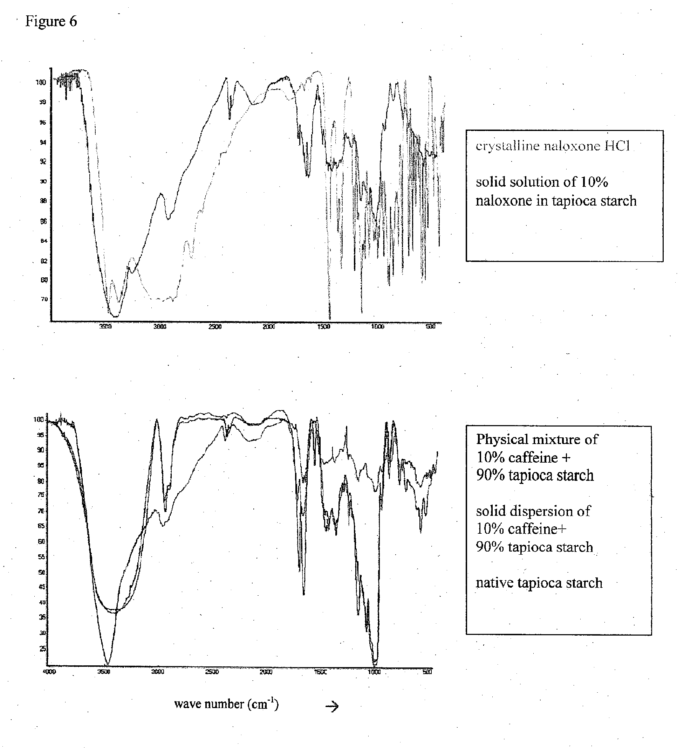

Solid solutions were then subjected to x-ray analysis. From Figures 5 A to 5 E one can clearly see, that even after 22 months there is no indication of a re-crystallisation of naloxone. In contrast, solid dispersions comprising caffeine as the pharmaceutically active compound embedded in amorphous starch show peaks indicative of the crystalline compound in the IR-spectrum. In Figure 6 the caffeine peaks are clearly detectable between 1.600 and 1.700 cm⁻¹, while the corresponding peaks for naloxone can not be detected within the amorphous solid solutions.

## Claims

1. A pharmaceutical dosage form being a solid solution wherein the dosage form comprises a least one pharmaceutically active compound in a polysaccharide matrix.

2. A pharmaceutical dosage form according to claim 1 wherein the dosage form is produced by extrusion of an overwet mixture of the at least one pharmaceutically active compound and at least one polysaccharide.

3. A pharmaceutical dosage form according to claim 2 wherein the overwet mixture of the pharmaceutically active compound and a polysaccharide or mixtures thereof comprises more than 15% (w/w) water, preferably more than 20% (w/w) water, even more preferably more than 25% (w/w) water and most preferably approximately 30% (w/w) water based on the weight of the overwet mixture.

4. A pharmaceutical dosage form according to one of claims 1 to 3 wherein the dosage form comprises starch, preferably potato-, corn- or tapioca-starch or mixtures thereof as matrix-forming materials.

5. A pharmaceutical dosage form according to one of claims 1 to 4 wherein the dosage form provides for a fast release of the active compound.

6. A pharmaceutical dosage form according to one of claims 1 to 4 wherein the dosage form provides for a sustained release of the active compound.

7. A pharmaceutical dosage form according to one of claims 1 to 6 wherein the matrix is a non-erosive diffusion matrix.

8. A pharmaceutical dosage form according to one of claims 1 to 7 wherein the matrix is amorphous or partially amorphous.

9. A pharmaceutical dosage form according to one of claims 1 to 8 wherein the matrix is produced with a single- or twin-screw extruder.

10. A pharmaceutical dosage form according to claim 9 wherein the matrix is produced with a counter- or co-rotating twin-screw extruder, preferably with a Leistritz Mikro 18 extruder.

11. A pharmaceutical dosage form according to one of claims 1 to 10 wherein the matrix comprises an analgesic opioid agonist and/or antagonist as pharmaceutically active compound(s).

12. A pharmaceutical dosage form according to claim 11 wherein the opioid agonist is selected from the group of morphine, oxycodone, hydromorphone, propoxyphene, nicomorphine, dihydrocodeine, diamorphine, papaveretum, codeine, ethylmorphine, phenylpiperidine and derivates thereof, methadone, dextropropoxyphene, buprenorphine, pentazocine, tilidine, tramadol and hydrocodone.

13. A pharmaceutical dosage form according to claim 12 wherein the opioid agonist is preferably oxycodone.

14. A pharmaceutical dosage form according to claim 11 wherein the opioid antagonist is selected from the group comprising naltrexone, naloxone, nalmefene, nalorphine, nalbuphine, naloxoneazinen, methylnaltrexone, ketylcyclazocine, norbinaltorphimine, naltrindol, 6-β-naloxol and 6-β-naltrexol.

15. A pharmaceutical dosage form according to claim 14 wherein the opioid antagonist is preferably naloxone.

16. A pharmaceutical dosage form according to one of the preceding claims wherein the matrix comprises oxycodone and naloxone as pharmaceutically active compounds.

17. A method of producing a pharmaceutical dosage form being a solid solution wherein the dosage form is produced by extrusion of an overwet mixture of at least one pharmaceutically active compound and at least one polysaccharide.

18. A method according to claim 17 wherein the overwet mixture of the pharmaceutically active compound and a polysaccharide or mixtures thereof comprises more than 15% (w/w) water, preferably more than 20% (w/w) water, even more preferably more than 25% (w/w) water and most preferably approximately 30% (w/w) water based on the weight of the overwet mixture.

19. A method according to claims 17 or 18 wherein the matrix comprises starch, preferably potato-, corn- or tapioca-starch or mixtures thereof as matrix-forming materials.

20. A method according to one of claims 17 to 19 wherein the matrix is produced with a single- or twin-screw extruder.

21. A method according to claim 20 wherein the matrix is produced with a counter- or co-rotating twin-screw extruder, preferably with a Leistritz Mikro 18 extruder.

22. A method according to one of claims 17 to 21 wherein the dosage form provides for a fast release of the active compound.

23. A method according to one of claims 17 to 21 wherein the dosage form provides for a sustained release of the active compound.

24. A method according to one of claims 17 to 23 wherein the matrix is a non-erosive diffusion matrix.

25. A method according to one of claims 17 to 24 wherein the matrix is amorphous or partially amorphous.

26. A method according to one of claims 17 to 24 wherein the matrix comprises an analgesic opioid agonist and/or antagonist as pharmaceutically active compound(s).
